Europäisches Patentamt

European Patent Office

Office européen des brevets

(19)

(11) **EP 0 667 845 B1**

(12) **EUROPEAN PATENT SPECIFICATION**

(45) Date of publication and mention
of the grant of the patent:
**14.01.1998 Bulletin 1998/03**

(21) Application number: **94925568.1**

(22) Date of filing: **07.09.1994**

(51) Int Cl.6: **C07C 17/154**, C07C 21/06

(86) International application number:
**PCT/GB94/01943**

(87) International publication number:
**WO 95/07250 (16.03.1995 Gazette 1995/12)**

(54) **OXYCHLORINATION CATALYST**

OXYCHLORIERUNGSKATALYSATOR

CATALYSEUR D'OXYCHLORATION

(84) Designated Contracting States:
**DE GB IT**

(30) Priority: **07.09.1993 GB 9318501**

(43) Date of publication of application:
**23.08.1995 Bulletin 1995/34**

(73) Proprietors:
• **EVC TECHNOLOGY AG**
**6300 Zug (CH)**
• **Hardman, Ray**
**Chester, Cheshire CH2 1NN (GB)**

(72) Inventors:
• **HARDMAN, Ray**
**Cheshire CH2 1NN (GB)**
• **CLEGG, Ian, Michael**
**Cheshire CW10 0AQ (GB)**

(74) Representative: **Votier, Sidney David**
**CARPMAELS & RANSFORD**
**43, Bloomsbury Square**
**London WC1A 2RA (GB)**

(56) References cited:
**FR-A- 2 285 359**

## Description

The present invention relates to a catalyst for the catalytic production of vinyl chloride monomer (VCM) from ethane via oxychlorination of ethane.

Most commercial processes for the production of VCM utilise ethylene and chlorine as the raw materials. Ethylene is in general chlorinated by contact with chlorine and a catalyst in liquid 1,2-dichloroethane. The dichloroethane is subsequently dehydrochlorinated at an elevated temperature to yield VCM and hydrogen chloride.

The use of ethylene as a starting material is a significant factor in the cost of producing VCM. In general, significant reductions in this cost can only be achieved by economies of scale since established processes are operating at close to maximum efficiency.

A further disadvantage of the use of ethylene is that the dehydrochlorination of the 1,2-dichloroethane intermediate yields hydrogen chloride. Disposal thereof is usually achieved by catalytic oxychlorination with ethylene in a further processing step to yield more 1,2-dichloroethane.

An alternative, known method for the production of VCM involves the use of ethane. The use of alternative hydrocarbon raw materials, of which ethane is the primary candidate, immediately addresses the issue of the cost of ethylene by substituting it with a cheaper alternative. Additionally, the chemistry of VCM production using alternative hydrocarbons may hold advantages. For example, VCM production can be achieved in a single step.

Three chemical approaches are known for the conversion of ethane to VCM. These are gas phase chlorination, catalysed oxidation or oxychlorination. Of these, a process based on oxychlorination is the most attractive:

$$C_2H_6 + Cl + O_2 \rightarrow C_2H_3Cl + \text{other products} + H_2O.$$

The chlorine source may be $Cl_2$, HCl or a chlorinated hydrocarbon. Where HCl is the source, the opportunity arises to utilise one of the intermediate products of VCM production from ethylene.

The production of VCM from ethane has not enjoyed commercial success. A number of attempts have been made, but the processes used have suffered from a number of drawbacks which, while inconvenient in a laboratory, become unacceptable when the process is applied on an industrial scale.

Early attempts, for example as reported in GB 1460688 (Lummus), utilised a Cu/K salt melt as the catalyst. These processes, although operable at relatively low temperatures (400°C), suffered from corrosion problems introduced by the molten catalyst which tends to damage the reactor vessel. The processes using salt melt catalyst have not gained commercial acceptance.

Alternative catalyst have been used in the art. For example, GB 2101596 (ICI) describes an unsupported silver/manganese catalyst. However, use of this catalyst has not been fully developed. US 4467127 (B.F. Goodrich) describes a fluidised bed catalyst based on iron. Such catalysts, however, have stability problems.

The most promising catalyst for oxychlorination of ethane appear to be fluidised bed catalysts based on copper. GB 1492945 (BP) describes such a catalyst, consisting of copper and potassium chlorides impregnated onto an alumina support, together with a cerium chloride component. However, it is not apparent that the precise formulation used by BP was successful, because the ethane feed used is diluted with in excess of 50% nitrogen gas, impractical in industrial applications, and comprehensive results are not reported.

Most by-products of the oxychlorination reaction are ignored. Furthermore, elevated temperatures (530°C) were employed which present difficulties in the industrial application.

SU 567714 also discloses a copper/potassium catalyst for the oxychlorination of ethane. However, the particular formulation used is only disclosed to be effective above 550°C, and up to 62.4% of the ethane reacted is unaccounted for in the quoted product spectrum.

GB 2009164 (Monsanto) describes, among others, a copper/potassium catalyst. However, the precise formulation used does not appear to be effective below 500°C. Furthermore, the quoted results are uninformative since it would appear that ethylene generation has been either ignored or confused with VCM generation. The % conversion to VCM is not given.

Accordingly, there remains a requirement in the art for an oxyhalogenation catalyst for the production of monohalogenated olefins from alkanes which is demonstrably applicable in a commercial environment. Such a catalyst should have acceptable performance at temperatures below 500°C.

According to a first aspect of the present invention, therefore, there is provided an oxyhalogenation catalyst capable of catalysing the production of a monohalogenated olefin from a alkane comprising a copper salt and an alkali metal salt deposited on an inert support, the catalyst containing copper and the alkali metal in an atomic ratio of 2:8.

The catalyst of the invention has been shown to provide favourable results in the oxychlorination of ethane when used at temperatures below 500°C.

The alkali metal may be lithium, potassium, sodium or rubidium. Preferably, however, the alkali metal is potassium.

The atomic ratio of the copper and potassium constituents quoted correspond to the weight percentages of 1.3% and 3.4%.

Preferably, a lanthanide salt may be used as an additional constituent. The preferred lanthanide is cerium, which may be added in atomic ratio between 0.1 and 5. Advantageously, a weight percentage of 0.74% of cerium is used, corresponding to an atomic ratio of 0.5.

Alternatively, an alkali earth metal may be used as an additional component. Preferred alkali earth metals are magnesium and calcium.

The inert carrier for the catalyst may be chosen from a number of supports known in the art which are readily available. For example, such supports include alumina, silica gel, silica-alumina, silica-magnesia, bauxite, magnesia, silicon carbide, titania, zirconium silicate and the like.

Preferably, the support is alumina. The preferred alumina is one having a low surface area to weight ratio. Preferably, this ratio is below $5m^2/g$ and advantageously it is around $1m^2/g$.

The catalyst may be used in a fixed bed or fluidised bed mode. Preferably, a fluidised bed is used. Fluidisation is accomplished by breaking up the catalyst into particles, which advantageously have a mean particle size of about 90 microns.

The preferred use for the catalyst is in oxychlorination reactions which yield VCM from ethane. However, it may be appreciated that the catalyst is also useful in, for example, oxybromination reactions and the like for the production of a variety of monohalogenated hydrocarbons.

When used for oxychlorination, the metal salts used in the catalyst are preferably metal chlorides. However, nitrates, carbonates and hydroxides may be used, which will be converted to chlorides, oxides or oxychlorides under reaction conditions.

The ethane feed used in the oxychlorination reaction may be of high purity, or may contain appreciable quantities of other hydrocarbons such as ethylene or methane, as in commercial or technical grade ethane supplies.

Oxygen may be supplied in the form of $O_2$ gas, or oxygen enriched air may be used.

Chlorine is provided in the form of $Cl_2$ gas, HCl or a chlorinated hydrocarbon, or mixtures thereof.

The ratios in which the various components of the reaction are advantageously mixed may be determined empirically. The preferred ratio for ethane to oxygen, however, is 1:0.25 to 1:1.4, and advantageously 1:0.75 to 1:1. The ratio of ethane to chlorine is preferably 1:0.5 to 1:5. All ratios are expressed on a molar basis.

The catalyst of the invention may be operated between the temperatures of 400 and 550°C. Preferably, however, the operating temperature is between 440 and 500°C and more preferably it is between 450 and 470°C.

The catalyst may be operated at a pressure in the range 0 to 30 BARA, preferably between 1 and 10 BARA.

The contact time of the reactants with the catalyst is preferably between 1 and 60 seconds and advantageously between 5 and 25 seconds.

The invention is described, for the purposes of illustration only, in the following examples.

<u>Examples 1 and 2</u>

A catalyst was prepared containing 1.3% copper and 3.4% potassium by evaporation of an aqueous solution of the metal chlorides onto an alumina carrier. To a 250 $cm^3$ sample of de-ionised water was added 20g of $CuCl_2.2H_2O$ and 35g of KCl. The resultant solution was added stepwise to 500g of catalyst carrier (Type SAHT-99, Production Chemicals Ltd.). This catalyst paste was dried by heating to 120°C for 24 hours and then sieved to break up agglomerates before use. When prepared, the catalyst was found to have a surface area of $1m^2/g$ and to have a mean particle size of 90 microns.

A catalyst charge of $400cm^3$ was loaded into a 50.8mm diameter Inconel fluidised bed reactor to give a bed length of approximately 40cm. The conversions and selectivity of the catalyst was measured using an on-line gas chromatograph. The reactor was heated electrically to the desired operating temperature and fed with a gas mixture of ethane, chlorine, nitrogen and oxygen and gave results as detailed in Table 1 example 1. The experiment was then repeated except that hydrogen chloride was substituted for chlorine and a higher flow of oxygen was used to account for increased stoichiometric requirement for oxygen - the results are shown as example 2, table 1. The use of hydrogen chloride instead of chorine resulted in a higher burning rate and a lower ethane conversion rate at a slightly lower oxygen conversion rate.

TABLE 1

|  |  | EXAMPLE 1 | EXAMPLE 2 |
|---|---|---|---|
| CONDITIONS | TEMP. (°C) | 478.0 | 480.7 |
|  | PRESS. (BARG) | 0.1 | 0.1 |

TABLE 1   (continued)

| | | EXAMPLE 1 | EXAMPLE 2 |
|---|---|---|---|
| REACTANT FLOWS IN (LITRES PER HOUR) | $Cl_2$ | 21.1 | 0 |
| | HCl | 0.0 | 48.0 |
| | $C_2H_4$ | 37.5 | 37.5 |
| | $N_2$ | 42.0 | 18.0 |
| | $O_2$ | 30.0 | 42.0 |
| PRODUCT FLOWS OUT (LITRES PER HOUR) | $O_2$ | 1.6 | 2.9 |
| | $N_2$ | 42.0 | 18.0 |
| | CO | 0.2 | 0.9 |
| | $CO_2$ | 4.0 | 4.6 |
| | $C_2H_4$ | 5.8 | 5.0 |
| | $C_2H_6$ | 2.0 | 3.8 |
| | $C_2H_2Cl$ | 11.4 | 10.1 |
| | $C_2H_5Cl$ | 4.9 | 4.7 |
| | $C_2H_2Cl_2$ (A) | 2.8 | 3.3 |
| | $C_2H_4Cl_2$ (B) | 0.6 | 0.6 |
| | $C_2H_2Cl_2$ (C) | 4.5 | 3.4 |
| | $C_2HCl_3$ | 0.2 | 0.3 |
| | $C_2Cl_4$ | 2.0 | 2.0 |
| | $C_2H_2Cl_2$ (D) | 0.2 | 0.2 |
| | UNKOWNS | 0.1 | 0.1 |
| | $CHCl_3$ | 0.2 | 0.3 |
| | $CCl_4$ | 0.5 | 0.9 |
| CONVERSION % | $O_2$ | 94.5 | 93.2 |
| | $C_2H_4$ | 94.8 | 89.9 |
| YIELD ON ETHANE REACTED | $C_2H_3Cl$ | 32.2 | 30.0 |
| | $C_2H_5Cl$ | 13.9 | 13.8 |
| | $C_2H_4$ | 16.3 | 14.8 |
| | $C_2H_2Cl_2$ (A) | 7.9 | 9.9 |
| | $C_2H_2Cl_2$ (D) | 0.5 | 0.5 |
| | $C_2HCl_3$ | 0.6 | 0.9 |
| | $C_2Cl_4$ | 5.9 | 8.4 |
| | $C_2H_4Cl_2$ | 14.5 | 12.0 |
| | $CO + CO_2$ | 5.9 | 8.4 |
| | CT (SEC) | 4.6 | 4.1 |
| | SLV (CM/SEC) | 4.4 | 5.0 |
| (NOTES: A-All 3 isomers, B-1, 1 isomer, C - 1, 2 isomer, D -1, 1,2 isomer) | | | |

Examples 3 to 5

A further charge of the catalyst detailed above was prepared and a sample of a copper, potassium and cerium catalyst was also prepared. This was carried out in the same manner except cerium chloride was added to give a metal content of 0.74% by weight. The Cu/K catalyst was fed with a mixture of ethane, hydrogen chloride, oxygen and nitrogen. This experiment was then repeated except that the Cu/K/Ce catalyst was used. Both these sets of results are shown as example 3, table 2. This comparison of the conversion and selectivities of these two catalyst formulations was repeated under two further sets of conditions at different feedstock ratios (shown as examples 4 and 5, table 2). Under all three sets of conditions, the incorporation of cerium into the catalyst formulations results in a significant beneficial effect: the burning rate is reduced and the conversion rate of ethane is raised.

TABLE 2

| | | EXAMPLE 3 | | EXAMPLE 4 | | EXAMPLE 5 | |
|---|---|---|---|---|---|---|---|
| | | Cu/K | Cu/K/Ce | Cu/K | Cu/K/Ce | Cu/K | Cu/K/Ce |
| CONDITIONS | TEMP. (°C) | 470.6 | 466.6 | 473.5 | 472.7 | 477.2 | 477.4 |
| | PRESS. (BARG) | 0.03 | 0.04 | 0.03 | 0.05 | 0.03 | 0.06 |
| REACTANT FLOWS IN (LITRES PER HOUR) | $Cl_2$ | 0 | 0 | 0 | 0 | 0 | 0 |
| | HCl | 60 | 60 | 60 | 60 | 60 | 60 |
| | $C_2H_4$ | 30 | 30 | 30 | 30 | 48 | 48 |
| | $N_2$ | 21.7 | 22 | 21.7 | 22 | 5.8 | 0 |
| | $O_2$ | 30 | 30 | 46 | 46 | 48 | 48 |
| PRODUCT FLOWS OUT (LITRES PER HOUR) | $O_2$ | 1.3 | 0.6 | 5.1 | 2.6 | 1.5 | 1.2 |
| | $N_2$ | 21.7 | 22.0 | 21.7 | 22.0 | 5.8 | 0.0 |
| | CO | 0.8 | 0.7 | 1.4 | 0.4 | 1.6 | 0.0 |
| | $CO_2$ | 1.8 | 0.9 | 3.7 | 3.4 | 4.2 | 3.8 |
| | $C_2H_4$ | 3.9 | 4.4 | 1.7 | 1.0 | 8.4 | 9.0 |
| | $C_2H_6$ | 3.2 | 1.6 | 1.5 | 0.7 | 5.4 | 3.6 |
| | $C_2H_2Cl$ | 7.7 | 8.6 | 4.8 | 4.7 | 12.4 | 13.6 |
| | $C_2H_5Cl$ | 3.6 | 3.2 | 2.5 | 1.6 | 5.8 | 6.2 |
| | $C_2H_2Cl_2$ (A) | 2.6 | 3.3 | 6.8 | 10.4 | 2.9 | 3.2 |
| | $C_2H_4Cl_2$ (B) | 0.5 | 0.5 | 0.6 | 0.3 | 0.6 | 0.7 |
| | $C_2H_2Cl_2$ (C) | 2.4 | 4.2 | 1.4 | 1.7 | 4.2 | 6.7 |
| | $C_2HCl_3$ | 0.2 | 0.4 | 1.7 | 2.3 | 0.3 | 0.2 |
| | $C_2Cl_4$ | 1.5 | 0.0 | 2.6 | 0.1 | 1.4 | 0.1 |
| | $C_2H_2Cl_2$ (D) | 0.0 | 2.4 | 0.1 | 4.5 | 0.2 | 2.4 |
| | UNKOWNS | 0.0 | 0.2 | 0.1 | 0.3 | 0.2 | 0.2 |
| | $CHCl_3$ | 0.2 | 0.2 | 0.4 | 0.4 | 0.5 | 0.4 |
| | $CCl_4$ | 0.7 | 0.4 | 1.4 | 1.2 | 1.0 | 0.9 |
| CONVERSION % | $O_2$ | 95.6 | 98.1 | 89.0 | 94.4 | 96.8 | 97.6 |
| | $C_2H_4$ | 89.3 | 94.8 | 95.1 | 97.8 | 88.8 | 92.6 |

TABLE 2   (continued)

| | | EXAMPLE 3 | | EXAMPLE 4 | | EXAMPLE 5 | |
|---|---|---|---|---|---|---|---|
| | | Cu/K | Cu/K/Ce | Cu/K | Cu/K/Ce | Cu/K | Cu/K/Ce |
| YIELD ON ETHANE REACTED | $C_2H_3Cl$ | 28.8 | 30.3 | 17.0 | 16.1 | 29.0 | 30.7 |
| | $C_2H_5Cl$ | 13.4 | 11.1 | 8.9 | 5.4 | 13.7 | 13.9 |
| | $C_2H_4$ | 14.6 | 15.5 | 5.8 | 3.4 | 19.7 | 20.4 |
| | $C_2H_2Cl_2$ (A) | 10.9 | 11.8 | 26.5 | 35.9 | 7.3 | 7.3 |
| | $C_2H_2Cl_2$ (D) | 0.1 | 8.7 | 0.5 | 15.7 | 0.5 | 5.5 |
| | $C_2HCl_3$ | 0.8 | 1.6 | 6.5 | 7.9 | 0.6 | 0.4 |
| | $C_2Cl_4$ | 6.0 | 0.1 | 10.1 | 0.4 | 3.4 | 0.2 |
| | $C_2H_4Cl_2$ | 12.0 | 16.9 | 7.7 | 7.2 | 12.0 | 16.7 |
| | $CO + CO_2$ | 5.3 | 2.9 | 9.8 | 6.6 | 7.2 | 4.3 |
| | CT (SEC) | 4.1 | 4.2 | 3.7 | 3.8 | 3.6 | 3.8 |
| | SLV (CM/SEC) | 5.0 | 4.9 | 5.5 | 5.4 | 5.7 | 5.4 |
| (NOTES: A-All 3 isomers, B-1, 1 isomer, C - 1, 2 isomer, D -1, 1,2 isomer) | | | | | | | |

Examples 6 to 22

Using Cu/K/Ce catalyst formulated in the same manner as detailed in example 2, the reactor was fed with a mixture of ethane, oxygen, 1,2 dichloroethane and nitrogen. Table 3 presents the results of a series of experiments under different temperatures and feedstock ratios. A range of conversions of ethane and oxygen are evident in the results as well as a range of reaction selectivities. The most attractive results are achieved at high temperature using a low flow of oxygen (example 13).

Examples 23 to 25

Again using the Cu/K/Ce formulation detailed in example 2, the reactor was fed with a mixture of ethane, oxygen, 1,2 dichloroethane, ethyl chloride and nitrogen: the results are shown as example 23, table 4. The experiment was then repeated but this time the reactor contained half the usual charge of catalyst and half of bare catalyst support. The catalyst was fluidised with nitrogen and held at 470°C for 8 hours before being fed with a mixture of ethane, oxygen, 1,2 dichloroethane, ethyl chloride and nitrogen and the results produced are displayed as example 24, table 4. The procedure was repeated again except using a catalyst that contained only 25% of the original charge of catalyst together with 75% of bare support. Results from this experiment are shown as example 25, table 4.

The results form this series show that the effect on feedstock conversion and selectivity is only measurable at between 50% and 25% of the initial metal concentration on the catalyst.

TABLE 3 A

| | Example | 6 | 7 | 8 | 9 | 10 | 11 | 12 |
|---|---|---|---|---|---|---|---|---|
| CONDITIONS | TEMP. (°C) | 490 | 540 | 453 | 449 | 451 | 511 | 524 |
| | PRESS. (BARG) | 0.83 | 0.86 | 0.76 | 0.75 | 0.78 | 0.83 | 0.87 |
| FLOWS IN (LITRES PER HOUR) | $C_2H_6$ | 29.90 | 29.9 | 30.4 | 30.3 | 50.7 | 30.2 | 51 |
| | $N_2$ | 143.6 | 143.5 | 143.5 | 143.5 | 143.7 | 142.3 | 142.3 |
| | $C_2H_4Cl_2$ (C) | 40 | 40 | 40 | 40 | 40 | 40 | 40.0 |
| | $O_2$ | 50.7 | 50.8 | 50.4 | 30.5 | 30.4 | 30.5 | 50.5 |

TABLE 3 A   (continued)

| | Example | 6 | 7 | 8 | 9 | 10 | 11 | 12 |
|---|---|---|---|---|---|---|---|---|
| FLOWS OUT (LITRES PER HOUR) | $O_2$ | 5.9 | 4.55 | 13.16 | 6.70 | 4.61 | 2.48 | 2.25 |
| | $N_2$ | 143.6 | 143.5 | 143.5 | 143.5 | 143.7 | 142.3 | 142.3 |
| | CO | 1.11 | 4.70 | 0.71 | 0.62 | 0.43 | 3.87 | 6.01 |
| | $CO_2$ | 15.85 | 14.25 | 13.32 | 8.61 | 9.94 | 4.71 | 16.61 |
| | $C_2H_4$ | 7.47 | 10.31 | 8.33 | 4.11 | 5.24 | 15.19 | 24.55 |
| | $C_2H_6$ | 3.76 | 3.47 | 6.64 | 13.04 | 28.83 | 5.24 | 14.70 |
| | $C_2H_3Cl$ | 21.42 | 23.17 | 10.75 | 9.42 | 7.25 | 28.62 | 22.74 |
| | $C_2H_3Cl$ | 3.67 | 2.09 | 7.43 | 8.62 | 10.99 | 2.37 | 3.61 |
| | $C_2H_2Cl_2$(A) | 4.52 | 4.61 | 0.94 | 0.50 | 0.23 | 2.54 | 0.98 |
| | $C_2H_4Cl_2$(B) | 0.29 | 0.23 | 0.48 | 0.38 | 0.27 | 0.17 | 0.10 |
| | $C_2H_4Cl_2$(C) | 20.09 | 11.05 | 30.10 | 32.49 | 35.18 | 15.48 | 15.21 |
| | $C_2HCl_3$ | 0.67 | 1.17 | 0.15 | 0.02 | 0.00 | 1.11 | 0.94 |
| | $C_2Cl_4$ | 0.54 | 1.12 | 1.07 | 0.62 | 0.28 | 0.44 | 0.11 |
| | $C_2H_3Cl_3$(D) | 2.33 | 0.76 | 0.30 | 0.19 | 0.00 | 0.58 | 0.21 |
| | Unkowns | 0.13 | 0.71 | 0.12 | 0.09 | 0.28 | 0.73 | 0.56 |
| | $CHCl_3$ | 0.25 | 0.14 | 0.21 | 0.17 | 0.11 | 0.09 | 0.00 |
| | $CCl_4$ | 0.30 | 0.29 | 0.17 | 0.18 | 0.12 | 0.23 | 0.00 |
| CONV. % | $O_2$ | 88.37 | 91.05 | 73.88 | 78.03 | 84.83 | 91.88 | 95.54 |
| | $C_2H_6$ | 87.44 | 88.41 | 78.15 | 56.96 | 43.14 | 82.66 | 71.17 |
| YIELD | $CO + CO_2$ | 32.43 | 35.86 | 29.51 | 26.72 | 23.72 | 17.18 | 31.16 |
| | CT SECS | 3.85 | 3.74 | 3.85 | 4.17 | 3.90 | 4.05 | 3.49 |
| | SLV CM/S | 5.30 | 5.45 | 5.29 | 4.89 | 5.22 | 5.03 | 5.84 |
| (NOTES: A-All 3 isomers, B-1, 1 isomer, C - 1, 2 isomer, D -1, 1. isomer) | | | | | | | | |

TABLE 3 B

| | Example | 13 | 14 | 15 | 16 | 17 | 18 | 19 |
|---|---|---|---|---|---|---|---|---|
| CONDITIONS | TEMP. (°C) | 510 | 514 | 453 | 457 | 518 | 518 | 491 |
| | PRESS. (BARG) | 0.76 | 0.83 | 0.73 | 0.75 | 0.76 | 0.77 | 0.79 |
| FLOWS IN (LITRES PER HOUR) | $C_2H_6$ | 50.8 | 30.3 | 30.2 | 50.8 | 30.7 | 49.6 | 40.2 |
| | $N_2$ | 101.8 | 143.7 | 144.9 | 107.8 | 103.7 | 82.1 | 122.2 |
| | $C_2H_4Cl_2$ (C) | 60.0 | 40.0 | 44.07 | 69.86 | 69.98 | 69.40 | 53.16 |
| | $O_2$ | 29.75 | 29.06 | 50.6 | 50.6 | 50.6 | 50.6 | 40.1 |

TABLE 3 B   (continued)

| Example | | | 13 | 14 | 15 | 16 | 17 | 18 | 19 |
|---|---|---|---|---|---|---|---|---|---|
| FLOWS OUT (LITRES PER HOUR) | | $O_2$ | 0.10 | 0.10 | 11.25 | 6.74 | 1.51 | 0.71 | 0.92 |
| | | $N_2$ | 101.8 | 143.7 | 144.9 | 107.8 | 103.7 | 82.1 | 122.2 |
| | | CO | 2.31 | 3.31 | 0.27 | 0.26 | 3.11 | 4.44 | 2.09 |
| | | $CO_2$ | 3.80 | 7.58 | 13.20 | 13.98 | 7.98 | 11.09 | 8.48 |
| | | $C_2H_4$ | 25.58 | 19.46 | 3.56 | 8.12 | 9.67 | 21.91 | 16.80 |
| | | $C_2H_6$ | 18.20 | 7.93 | 9.84 | 17.81 | 3.24 | 10.98 | 7.86 |
| | | $C_2H_3Cl$ | 37.70 | 21.84 | 11.32 | 17.51 | 40.66 | 40.28 | 31.99 |
| | | $C_2H_3Cl$ | 3.37 | 1.71 | 7.65 | 13.72 | 1.95 | 3.41 | 4.24 |
| | | $C_2H_2Cl_2(A)$ | 1.89 | 1.38 | 1.06 | 1.05 | 10.11 | 3.46 | 5.23 |
| | | $C_2H_4Cl_2(B)$ | 0.13 | 0.05 | 0.55 | 0.69 | 0.12 | 0.23 | 0.19 |
| | | $C_2H_4Cl_2(C)$ | 27.06 | 12.41 | 32.64 | 54.83 | 18.01 | 22.81 | 22.76 |
| | | $C_2HCl_3$ | 1.11 | 0.81 | 0.10 | 0.11 | 3.33 | 2.30 | 0.98 |
| | | $C_2Cl_4$ | 0.25 | 0.14 | 1.48 | 1.44 | 1.41 | 0.40 | 1.26 |
| | | $C_2H_3Cl_3(D)$ | 0.38 | 0.00 | 0.23 | 0.28 | 1.69 | 0.93 | 0.41 |
| | | Unkowns | 0.54 | 0.54 | 0.02 | 0.26 | 1.56 | 1.13 | 0.67 |
| | | $CHCl_3$ | 0.00 | 0.00 | 0.21 | 0.39 | 0.22 | 0.00 | 0.10 |
| | | $CCl_4$ | 0.00 | 0.00 | 0.25 | 0.29 | 0.38 | 0.16 | 0.30 |
| CONV. % | | $O_2$ | 99.66 | 99.66 | 77.78 | 86.68 | 97.01 | 98.60 | 97.70 |
| | | $C_2H_6$ | 64.17 | 73.82 | 67.41 | 64.94 | 89.46 | 77.86 | 80.45 |
| YIELD | | $CO + CO_2$ | 9.38 | 24.35 | 33.09 | 21.58 | 20.20 | 20.11 | 16.34 |
| | | CT SECS | 3.91 | 4.03 | 3.71 | 3.61 | 3.67 | 3.74 | 3.86 |
| | | SLV CM/S | 5.22 | 5.06 | 5.49 | 5.64 | 5.55 | 5.44 | 5.28 |
| (NOTES: A-All 3 isomers, B-1, 1 isomer, C - 1, 2 isomer, D -1, 1, 2 isomer) | | | | | | | | | |

TABLE 3 C

| Example | | 20 | 21 | 22 |
|---|---|---|---|---|
| CONDITIONS | TEMP. (°C) | 531 | 520 | 470 |
| | PRESS. (BARG) | 0.8 | 0.8 | 0.77 |
| FLOWS IN (LITRES PER HOUR) | $C_2H_6$ | 50.4 | 50.2 | 44.9 |
| | $N_2$ | 102.49 | 102.3 | 102.6 |
| | $C_2H_4Cl_2(C)$ | 44.47 | 57.60 | 64.00 |
| | $O_2$ | 50.1 | 30 | 35 |

TABLE 3 C   (continued)

|  |  | Example | 20 | 21 | 22 |
|---|---|---|---|---|---|
| FLOWS OUT (LITRES PER HOUR) |  | $O_2$ | 0.31 | 0.21 | 0.56 |
|  |  | $N_2$ | 102.5 | 102.3 | 102.6 |
|  |  | CO | 4.31 | 2.42 | 2.11 |
|  |  | $CO_2$ | 16.78 | 8.00 | 10.13 |
|  |  | $C_2H_4$ | 23.30 | 21.69 | 14.52 |
|  |  | $C_2H_6$ | 16.10 | 23.73 | 17.58 |
|  |  | $C_2H_3Cl$ | 23.32 | 24.46 | 21.29 |
|  |  | $C_2H_3Cl$ | 2.34 | 2.31 | 5.62 |
|  |  | $C_2H_2Cl_2$(A) | 1.56 | 2.14 | 3.19 |
|  |  | $C_2H_4Cl_2$(B) | 0.08 | 0.04 | 0.24 |
|  |  | $C_2H_4Cl_2$(C) | 9.65 | 20.77 | 35.46 |
|  |  | $C_2HCl_3$ | 1.11 | 0.89 | 0.44 |
|  |  | $C_2Cl_4$ | 0.15 | 0.15 | 0.60 |
|  |  | $C_2H_3Cl_3$(D) | 0.57 | 0.33 | 0.00 |
|  |  | Unkowns | 0.48 | 0.18 | 0.71 |
|  |  | $CHCl_3$ | 0.00 | 0.00 | 0.00 |
|  |  | $CCl_4$ | 0.00 | 0.00 | 0.14 |
| CONV. % |  | $O_2$ | 99.39 | 99.32 | 98.41 |
|  |  | $C_2H_6$ | 68.06 | 52.73 | 60.85 |
| YIELD |  | $CO + CO_2$ | 30.74 | 19.68 | 22.39 |
|  |  | CT SECS | 3.82 | 4.00 | 4.09 |
|  |  | SLV CM/S | 5.33 | 5.09 | 4.98 |
| (NOTES: A-All 3 isomers, B-1, 1 isomer, C - 1, 2 isomer, D -1, 1, 2 isomer) | | | | | |

TABLE 4

|  |  |  | EXAMPLE 23 | EXAMPLE 24 | EXAMPLE 25 |
|---|---|---|---|---|---|
| CONDITIONS | TEMP. (°C) |  | 490.10 | 491.4 | 490.1 |
|  | PRESS. (BARG) |  | 0.05 | 0.08 | 0.13 |
| REACTANT FLOWS IN (LITRES PER HOUR) | $C_2H_6$ |  | 35.30 | 35.43 | 35.40 |
|  | $N_2$ |  | 59.90 | 59.70 | 69.10 |
|  | $C_2H_4Cl_2$ (C) |  | 40.00 | 40.29 | 40.00 |
|  | $C_2H_5Cl$ |  | 8.82 | 8.78 | 12.24 |
|  | $O_2$ |  | 28.00 | 27.70 | 28.20 |

TABLE 4   (fortgesetzt)

| | | | EXAMPLE 23 | EXAMPLE 24 | EXAMPLE 25 |
|---|---|---|---|---|---|
| PRODUCT FLOWS OUT (LITRES PER HOUR) | $O_2$ | | 0.37 | 0.19 | 2.56 |
| | CO | | 0.29 | 1.07 | 1.47 |
| | $CO_2$ | | 2.24 | 1.96 | 2.58 |
| | $C_2H_4$ | | 18.35 | 17.32 | 18.55 |
| | $C_2H_6$ | | 8.21 | 8.07 | 9.20 |
| | $C_2H_3Cl$ | | 25.86 | 23.00 | 21.52 |
| | $C_2H_5Cl$ | | 8.58 | 9.15 | 11.78 |
| | $C_2H_2Cl_2$ (A) | | 1.48 | 1.09 | 0.95 |
| | $C_2H_4Cl_2$ (B) | | 0.35 | 0.46 | 0.59 |
| | $C_2H_4Cl_2$ (C) | | 19.47 | 21.71 | 23.86 |
| | $C_2HCl_3$ | | 0.27 | 0.21 | 0.13 |
| | $C_2Cl_4$ | | 0.35 | 0.21 | 0.14 |
| | $C_2H_3Cl_3$ (D) | | 0.86 | 0.80 | 0.67 |
| | UNKOWNS | | 0.18 | 0.00 | 0.10 |
| | $CHCl_3$ | | 0.12 | 0.15 | 0.24 |
| | $CCl_4$ | | 0.19 | 0.14 | 0.23 |
| CONVERSIONS % | $O_2$ | | 98.70 | 99.31 | 90.92 |
| | $C_2H_6$ | | 76.73 | 77.21 | 74.00 |
| YIELD ON ETHANE REACTED | $C_2H_3Cl$ | | 95.46 | 84.08 | 82.17 |
| | $CO + CO_2$ | | 4.68 | 5.55 | 7.74 |
| | $C_2H_3Cl_3$ (D) | | 3.17 | 2.93 | 2.37 |
| | $C_2HCl_3$ | | 0.99 | 0.78 | 0.49 |
| | $C_2Cl_4$ | | 1.29 | 0.76 | 0.53 |
| | CT SECS | | 3.28 | 3.47 | 3.38 |
| | SLV CM/S | | 6.21 | 5.87 | 6.03 |
| (NOTES: A-All 3 isomers, B-1, 1 isomer, C - 1, 2 isomer, D -1, 1, 2 isomer) | | | | | |

**Patentansprüche**

1. Oxyhalogenierungskatalysator, der befähigt ist, die Herstellung eines monohalogenierten Olefins aus einem Alkan zu katalysieren, welcher ein Kupfersalz und ein Alkalimetallsalz, welche auf einem inerten Träger abgeschieden sind, umfaßt, wobei der Katalysator Kupfer und ein Alkalimetall im Atomverhältnis 2:8 enthält.

2. Katalysator nach Anspruch 1, wobei das Alkalimetall Kalium ist.

3. Katalysator nach Anspruch 1 oder 2, welcher weiter ein Lanthanoidsalz umfaßt.

4. Katalysator nach Anspruch 1 oder 2, welcher weiter ein Erdalkalimetall umfaßt.

5. Katalysator nach Anspruch 3, wobei das Lanthanoid Cer ist.

6. Katalysator nach einem vorhergehenden Anspruch, wobei der inerte Träger Aluminiumoxid ist.

**7.** Katalysator nach Anspruch 6, wobei das Aluminiumoxid ein Verhältnis von Oberfläche zu Gewicht von unterhalb 5m$^2$/g aufweist.

**8.** Katalysator nach einem vorhergehenden Anspruch, welcher zwischen 450 und 470°C betriebsfähig ist.

**9.** Katalysator nach einem vorhergehenden Anspruch, welcher befähigt ist, die Oxychlorierung von Ethan zu katalysieren, um VCM zu bilden.

**10.** Katalysator nach Anspruch 9, wobei die Metallsalze Metallchloride sind.

## Claims

**1.** An oxyhalogenation catalyst capable of catalysing the production of a monohalogenated olefin from an alkane comprising a copper salt and an alkali metal salt deposited on an inert support, the catalyst containing copper and an alkali metal in the atomic ratio 2:8.

**2.** The catalyst of claim 1 wherein the alkali metal is potassium.

**3.** The catalyst of claim 1 or claim 2 further comprising a lanthanide salt.

**4.** The catalyst of claim 1 or claim 2 further comprising an alkali earth metal.

**5.** The catalyst of claim 3 wherein the lanthanide is cerium.

**6.** The catalyst of any preceding claim wherein the inert support is alumina.

**7.** The catalyst of claim 6 wherein the alumina has a surface area to weight ratio below 5m$^2$/g.

**8.** The catalyst of any preceding claim which is operable between 450 and 470°C.

**9.** The catalyst of any preceding claim which is capable of catalysing the oxychlorination of ethane to form VCM.

**10.** The catalyst of claim 9 wherein the metal salts are metal chlorides.

## Revendications

**1.** Catalyseur d'oxyhalogénation apte à catalyser la production d'une oléfine monohalogénée à partir d'un alcane, comprenant un sel de cuivre et un sel de métal alcalin déposé sur un support inerte, le catalyseur contenant du cuivre et un métal alcalin dans le rapport atomique de 2:8.

**2.** Catalyseur selon la revendication 1, dans lequel le métal alcalin est le potassium.

**3.** Catalyseur selon la revendication 1 ou 2, comprenant en outre un sel de lanthanide.

**4.** Catalyseur selon la revendication 1 ou 2, comprenant en outre un métal alcalino-terreux.

**5.** Catalyseur selon la revendication 3, dans lequel le lanthanide est le cérium.

**6.** Catalyseur selon l'une quelconque des revendications précédentes, dans lequel le support inerte est l'alumine.

**7.** Catalyseur selon la revendication 6, dans lequel l'alumine possède un rapport aire de surface/poids inférieur à 5 m$^2$/g.

**8.** Catalyseur selon l'une quelconque des revendications précédentes, qui peut être mis en oeuvre entre 450 et 470°C.

9. Catalyseur selon l'une quelconque des revendications précédentes, qui est apte à catalyser l'oxychloration d'éthane pour former du VCM (monomère de chlorure de vinyle).

10. Catalyseur selon la revendication 9, dans lequel les sels métalliques sont des chlorures métalliques.